# FASCICULE DE BREVET EUROPEEN

(11) **EP 0 906 464 B1**
(45) Date de publication et mention de la délivrance du brevet: **09.01.2002**
(21) Numéro de dépôt: 97929370.1
(22) Date de dépôt: 17.06.1997
(51) Int. Cl.: D06L 1/00, C11D 11/00, D06M 23/10, A61K 9/70, A61K 47/34

(54) **PROCEDE DE FABRICATION ET DE TRAITEMENT DES TEXTILES**
VERFAHREN ZUR HERSTELLUNG UND BEHANDLUNG VON TEXTILIEN
METHOD FOR MANUFACTURING AND TREATING TEXTILES

(30) Priorité: 18.06.1996 FR 9607720
(43) Date de publication de la demande: 07.04.1999
(73) Titulaire: BIOLAND Société à Responsabilité Limitée, F-31100 Toulouse (FR)
(72) Inventeur: PODDEVIN, Nicolas, 31770 Colomiers (FR); FAGES, Jacques, F-81000 Albi (FR); GUIDOIN, Robert, F-49150 Bauge (FR)
(74) Mandataire: Lassiaille, Christian Michel
(86) Numéro de dépôt international: FR9701093
(87) Numéro de publication internationale: WO9748848

(56) Documents cités:
- EP-A- 0 679 753
- EP-B- 0 464 163
- EP-B- 0 518 653
- EP-B- 0 530 949
- DE-A- 3 904 514
- DE-A- 4 004 111
- DE-A- 4 238 620
- DE-A- 4 314 869
- DE-A- 4 333 221
- DE-A- 4 336 941
- US-A- 4 012 194

## Description

L'invention concerne la fabrication et le traitement d'une structure textile contenant au moins un polymère de la famille des poly(α-hydroxy-acides), c'est-à-dire comprenant des fibres textiles incorporant une portion d'au moins un polymère de la famille des poly(α-hydroxy-acides).

Dans toute la présente demande, l'expression "structure textile" couvre non seulement toutes les structures textiles obtenues par assemblage de fils ou filés de fibres (tissu, non-tissé, tresse, tricot, tulle, composite...), à l'état morcelé ou en nappe ou même en bobine, mais également les fils, filés, filaments, cordes, câbles eux-mêmes, aussi désignés ci-après de façon générique par le terme "fils".

Dans l'industrie textile, on assemble mécaniquement des fibres textiles lors d'au moins une étape mécanique d'assemblage telle que la filature (ensemble des opérations d'assemblage des fibres entre elles pour l'obtention d'un fil) ; le tressage, le tricotage et le tissage (assemblage des fils par entrelaçage et/ou entrebouclage...) ; le feutrage, le collage, l'agglomération des fibres et/ou des fils et/ou de plusieurs étoffes entre elles ou à d'autres produits (non tissé, composite...).

Après ces étapes d'assemblage, les pièces dites "tombées de métier" ou écrues, subissent des traitements de finition (désencollage, blanchiment, teinture, impression, apprêts...).

A l'issue de toutes ces étapes, la structure textile obtenue présente les formes et l'aspect désirés, mais incorpore de nombreuses impuretés indésirables et inutiles, emprisonnées dans la structure, et qui n'entrent pas dans la composition des fibres ou de la structure. Parmi ces impuretés, on peut citer : les impuretés incorporées dans les fibres lors de l'obtention ou de la fabrication des fibres elles-mêmes ; les adjuvants et les impuretés incorporés volontairement (par exemple des adjuvants d'ensimage ou antistatiques) ou non lors desdites étapes d'assemblage ; les contaminants organiques ou les micro-organismes...

Compte-tenu de la grande difficulté qu'il y a à éliminer l'ensemble de ces impuretés incorporées au coeur même de la structure et des fibres, on se résigne dans la plupart des applications à utiliser ces structures textiles ainsi contaminées.

Dans le cas particulier où une purification s'impose, par exemple pour la fabrication de matériaux implantables en structure de fibres textiles (ligaments artificiels, patch, membranes artificielles, prothèses vasculaires...), on procède à une succession d'étapes complexes, multiples, de natures très différentes (adaptées à l'élimination de chaque type d'impuretés), et sophistiquées, de lavage, nettoyage, désensimage, désinfection, élimination des solvants, neutralisation...

Tous ces traitements consistent à utiliser des produits actifs tels que des détergents, des désinfectants, des solvants puissants et nocifs qui peuvent eux-mêmes rester à l'état de traces dans la structure et sont dangereux pour les manipulateurs. En outre, ces traitements sont longs, coûteux et sont souvent polluants du fait de l'utilisation de ces produits puissants et nocifs.

Malgré tous ces traitements, il n'est pas rare de constater que les structures restent quelque peu contaminées. Par exemple, il a été démontré que des réactions immunitaires négatives peuvent être dues à des contaminations de surface de prothèses vasculaires implantées dans des conditions stériles parfaites.

Tel est en particulier le cas des structures à fibres textiles biorésorbables en polymères fragiles qui ne supportent pas les solvants, les détergents ou les désinfectants, ni les traitements thermiques. On renonce donc jusqu'à maintenant à utiliser ces fibres pour la fabrication de pièces nécessitant une grande pureté.

Or, ces fibres textiles fragiles possèdent parfois, par ailleurs, des propriétés particulières, recherchées pour des applications précises. Tel est le cas en particulier des fibres contenant au moins un poly(α-hydroxy-acide) qui sont biocompatibles et/ou biodégradables, et notamment des fibres d'acide polyglycolique (PGA) qui présentent d'excellentes propriétés de biorésorption.

Néanmoins, il était considéré jusqu'à maintenant que les polymères de la famille des poly(α-hydroxy-acides) ne peuvent pas être soumis à des traitements physico-chimiques intenses, compte tenu de leur grande fragilité. En particulier, il est connu que les polymères de la famille des poly(α-hydroxy-acides) sont solubles dans les fluides à l'état supercritique (cf. par exemple "Formation of bioredible polymeric microspheres and microparticles by rapid expansion of supercritical solutions", J. W. Tom and P.G. Debenedetti, Biotechnol. Prog, 1991, 7, 403-411 ; EP-0464163...). En outre, les traitements de purification appliqués à ces matériaux ont pour conséquence de les fragiliser, même si ces traitements restent toujours imparfaits. Le besoin se fait donc sentir pour de nombreuses applications d'un procédé de traitement permettant de purifier complètement les structures textiles à base de polymères de la famille des poly(α-hydroxy-acides), et ne dégradant pas les caractéristiques mécaniques de la structure.

Or, les inventeurs ont déterminé de façon surprenante et en contradiction avec les enseignements antérieurs, qu'une structure textile contenant au moins un polymère de la famille des poly(α-hydroxy-acides) peut être soumise à un traitement par une composition de fluide(s) à l'état supercritique sans aucun dommage pour la structure. Les inventeurs ont aussi montré que ce traitement permet alors de purifier intégralement en une seule étape une telle structure textile, non seulement en préservant l'intégrité physico-chimique de la structure mais même, de façon encore plus surprenante, en améliorant ses caractéristiques mécaniques.

Dans cette mesure, l'invention vise à proposer un procédé de fabrication et un procédé de traitement d'une structure textile contenant au moins un polymère de la famille des poly(α-hydroxy-acides) permettant d'obtenir une structure textile pure et/ou de caractéristiques mécaniques renforcées.

L'invention vise plus particulièrement à proposer un traitement efficace, simple et peu coûteux pour la purification des structures textiles contenant au moins un polymère de la famille des poly(α-hydroxy-acides) en assurant l'élimination des différents composés physico-chimiques indésirables et, simultanément, celle des différents contaminants organiques et biochimiques (micro-organismes), tout en préservant l'intégrité physico-chimique de la structure.

Ainsi, l'invention vise à proposer un procédé de traitement de purification totale en une seule étape de structures textiles contenant au moins un polymère de la famille des poly(α-hydroxy-acides).

L'invention vise aussi en particulier à proposer un traitement des structures textiles contenant au moins un polymère de la famille des poly(α-hydroxy-acides) permettant de renforcer la résistance mécanique de ces structures.

La famille des poly(α-hydroxy-acides) comprend les polymères de formule générale : 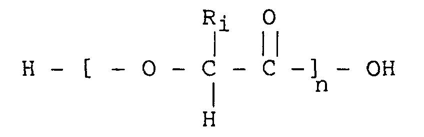 où Rᵢ est un hydrogène ou un méthyle. Cette famille comprend non seulement les homopolymères répondant à cette formule, mais aussi les copolymères correspondants et les mélanges de ces homopolymères et copolymères. Parmi ces polymères, on peut citer l'acide polyglycolique (PGA), et les acides polylactiques L-PLA, D-PLA, DL-PLA, et les copolymères poly(lactide-coglycolite) PLA/GA.

Tous ces polymères présentent des propriétés semblables. En particulier, ils sont biocompatibles et biodégradables. Par exemple, un implant de PGA est biorésorbable en une durée de l'ordre de 1 à 6 mois. En outre, à partir d'une certaine valeur de poids moléculaire qui dépend de chaque polymère ou de chaque composition polymérique, ils peuvent être filés par extrusion et permettent la fabrication de structures textiles.

L'invention vise aussi à proposer un tel procédé qui permette d'obtenir des structures textiles contenant notamment des structures textiles biodégradables ou biorésorbables pouvant rentrer dans la composition des implants (pièces implantables) pour l'homme ou l'animal.

Ainsi, l'invention vise en particulier à proposer un procédé de traitement avant implantation de structures textiles -notamment de fils ou de structure tissée et/ou tricotée et/ou tressée et/ou agglomérée (non tissé, composite)...- , contenant au moins un polymère de la famille des poly(α-hydroxy-acides), qui garantisse la pureté et la stérilité de ces structures, en préservant leur intégrité physico-chimique et mécanique.

L'invention vise aussi à proposer un procédé de traitement qui n'impose pas de placer les pièces à haute température, et qui donc peut être mis en oeuvre à basse température (notamment à moins de 60° C).

L'invention vise aussi à proposer un procédé de traitement qui soit extrêmement propre, ne produise pas d'effluents polluants ou toxiques, et ne soit pas dangereux pour les manipulateurs.

L'invention vise aussi à proposer un tel procédé qui augmente les possibilités d'utilisation des fibres textiles contenant au moins un polymère de la famille des poly(α-hydroxy-acides), notamment pour l'obtention de matériaux ou de pièces biorésorbables implantables dans le corps humain ou animal.

Pour ce faire, l'invention concerne un procédé conforme à la revendication 1.

Le poids moléculaire des polymères poly(α-hydroxy-acides) entrant dans la composition des fibres permettant l'obtention de la structure est adapté -notamment suffisant- pour permettre l'assemblage -notamment le filage par extrusion à chaud- des fibres. En effet, en particulier, le filage par extrusion à chaud de fibres de ces polymères n'est possible avec une qualité satisfaisante qu'à partir d'une certaine valeur de poids moléculaire qui dépend de la composition chimique exacte des polymères, et du procédé d'assemblage mis en oeuvre.

Dans le cas du PGA, le poids moléculaire du polymère doit être classiquement supérieur à 30 000, notamment de l'ordre de 120 000, pour un filage par extrusion à chaud de fils destinés au tissage.

On rappelle que les fluides à l'état supercritique peuvent être définis comme des gaz placés dans des conditions de température et de pression telles que leurs propriétés sont intermédiaires entre celles des gaz et celles des liquides. On les nomme encore "gaz denses" ou "liquides expansés". Pour un corps chimique donné, le point précis du diagramme température-pression pour lequel les deux phases liquide et vapeur n'en forment plus qu'une est appelé point critique. Au-delà de cette température critique (Tc) et de cette pression critique (Pc), le fluide est en l'état dit "supercritique".

Différents fluides à l'état supercritique peuvent être utilisés dans le cadre de l'invention. Il est à noter à cet égard que si les fluides supercritiques sont surtout connus pour leur fonction d'extraction des matières organiques à partir des végétaux ou d'organes, (par exemple US-4749522), l'invention a mis en évidence que ces fluides permettent simultanément de laver, de nettoyer, de désensimer et de désinfecter les structures textiles contenant au moins un polymère de la famille des poly(α-hydroxy-acides) avec une très grande efficacité, tout en ayant, de façon surprenante, une grande neutralité physico-chimique, et mécanique vis-à-vis de cette structure et des polymères de la famille des poly(α-hydroxy-acides), et même en renforçant la résistance mécanique de la structure.

Avantageusement et selon l'invention, on choisit du dioxyde de carbone à l'état supercritique à titre de composition de fluide(s) à l'état supercritique.

Avantageusement et selon l'invention, on place la pièce au contact d'un courant de dioxyde de carbone supercritique pendant une durée comprise entre 1 h et 24h, notamment de l'ordre de 12 h.

L'invention s'étend aussi à un procédé de fabrication conforme à la revendication 4.

Avantageusement et selon l'invention, on réalise la structure par assemblage de fils obtenus par extrusion de fibres contenant au moins un polymère de la famille des poly(α-hydroxy-acides).

Selon l'invention, la structure textile contient au moins une proportion sensible -notamment plus de 1 %- de poly(α-hydroxy-acides). Autrement dit, la structure textile comprend des fibres contenant une proportion sensible d'au moins un polymère de la famille des poly(α-hydroxy-acides) dont le poids moléculaire est adapté pour permettre l'obtention de ladite structure.

L'invention est applicable quelle que soit la proportion en polymère(s) de la famille des poly(α-hydroxy-acides), notamment de fibres de ce(s) polymère(s), contenue dans la structure textile. Néanmoins, avantageusement et selon l'invention, on réalise la structure avec des fils obtenus par extrusion de fibres de poly(α-hydroxy-acide(s)) -notamment de PGA-, c'est-à-dire que la structure est formée à 100 % de fibres comprenant 100 % de poly (α-hydroxy-acide(s)) -notamment de PGA-.

Avantageusement dans un procédé de fabrication selon l'invention, on réalise le conditionnement final de la structure après ladite étape de traitement.

Avantageusement et selon l'invention, on soumet la structure à une et une seule étape de traitement après toutes les étapes d'assemblage permettant d'obtenir la structure. De préférence, avantageusement et selon l'invention, on soumet la pièce au procédé de traitement immédiatement après avoir effectué toutes les étapes d'assemblage et de finition pour l'obtention de ladite structure, et sur le même site. Ainsi, avantageusement et selon l'invention, on effectue ladite étape de traitement immédiatement après la fabrication de la pièce.

L'invention s'étend aussi à une structure textile biorésorbable contenant au moins un polymère de la famille des poly(α-hydroxy-acides) obtenue par au moins une étape mécanique d'assemblage (filature et/ou tressage et/ou tricotage et/ou tissage et/ou agglomération...) de fibres textiles au moins partiellement formées de fibres contenant une proportion d'au moins un polymère de la famille des poly(α-hydroxy-acides) dont le poids moléculaire est adapté pour permettre l'obtention de ladite structure, et ayant été obtenue par un procédé selon l'invention. Avantageusement et selon l'invention, la structure est formée d'un assemblage de fils obtenus par extrusion de fibres contenant -notamment constituées par- au moins un polymère de la famille des poly(α-hydroxy-acides) dont le poids moléculaire est adapté pour permettre l'obtention de ladite structure, notamment un assemblage tissé et/ou tricoté et/ou tressé et/ou aggloméré de fils. Avantageusement et selon l'invention, la structure est formée d'une pièce tissée biorésorbable de fils de PGA. Une structure textile selon l'invention est à la fois exempte d'impuretés et de caractéristiques physico-chimiques et mécaniques inaltérées, voire même renforcées. Une telle structure n'avait pas encore pu être obtenue dans l'art antérieur.

En particulier, l'invention concerne ainsi une pièce stérile et pure comprenant une structure textile de fibres contenant au moins un polymère de la famille des poly(α-hydroxy-acides), selon l'invention qui est suffisamment pure pour pouvoir être implantable chez l'homme ou l'animal, y compris sur des sites d'implantation particulièrement sensibles. Les caractéristiques mécaniques et physico-chimiques de la pièce sont semblables ou même améliorées par rapport à celles d'une pièce impure non traitée conformément à l'invention.

L'invention s'étend aussi à l'application d'un procédé de traitement selon l'invention pour le traitement avant leur implantation dans le corps humain ou animal, d'implants à structure textile selon l'invention, notamment d'implants en forme de fils ou d'implants à structure tissée et/ou tricotée et/ou tressée et/ou agglomérée, contenant au moins un polymère de la famille des poly(α-hydroxy-acides) biorésorbables.

L'invention s'étend aussi à l'application d'un procédé de fabrication selon l'invention pour la fabrication d'implants à structure textile contenant au moins un polymère de la famille des poly(α-hydroxy-acides).

L'invention concerne aussi un procédé de traitement, un procédé de fabrication, une structure, et une pièce ou un implant, caractérisés en combinaison, par tout ou partie des caractéristiques ci-dessus ou ci-après mentionnées.

D'autres caractéristiques et avantages de l'invention apparaîtront des exemples suivants.

### EXEMPLE 1 :

On fabrique une pièce de structure textile tissée selon une armure satin avec des fils formés de fibres d'acide polyglycolique (PGA 100) selon un duitage de 25 fils/cm et avec un nombre de 85 fils/cm. Il est à noter que ces fibres d'acide polyglycolique sont des fibres biorésorbables et qu'elles sont particulièrement sensibles et fragiles aux traitements de purification traditionnels.

On soumet un fragment de cette pièce à un traitement selon l'invention en la plaçant au contact d'un courant de dioxyde de carbone supercritique sous une pression de 2,8.10⁷ Pa pendant 15 heures à 35° C avec un débit moyen de 54,6 kg/h de CO₂ supercritique. On constate à l'examen visuel et au toucher que la pièce n'est pas altérée du point de vue de son aspect, de sa souplesse et de sa résistance.

Une analyse de contamination est ensuite effectuée sur le fragment de pièce ainsi traité. On constate l'absence totale de micro-organismes aérobies et mésophiles, et de levure et moisissures.

Un contrôle de stérilité effectué sur le fragment de pièce démontre qu'il est stérile après le traitement.

Des essais mécaniques de résistance à la rupture en traction sont effectués sur le fragment de pièce traité selon l'invention, sur un fragment témoin non traité de la même pièce, et sur un fragment de la même pièce stérilisé aux rayons γ et non traité au CO₂ supercritique.

Les résultats sont les suivants :
. fragment témoin non traité : 732N - 727,5N - 730N, soit une valeur moyenne de 729,8N,
. fragment stérilisé aux rayons γ : 617,5N - 614N - 625N, soit une valeur moyenne de 618,8N,
. fragment traité selon l'invention : 762N - 732,5N - 735N, soit une valeur moyenne de 743,1N.
   Ainsi, contrairement à la stérilisation aux rayons γ qui diminue de plus de 15 % la résistance de la structure textile, le traitement selon l'invention augmente cette résistance d'environ 2 %. Ce phénomène ne trouve pas d'explication claire alors que le PGA en masse (brut non divisé) est normalement soluble dans le dioxyde de carbone supercritique, et que l'on s'attendait donc à ce que la résistance mécanique soit au contraire considérablement dégradée.

### EXEMPLE 2 :

On fabrique deux fragments de pièce de tissu d'armure satin comme indiqué dans l'exemple 1. Le premier est utilisé brut à la sortie du métier, tandis que le second et soumis au traitement au CO₂ supercritique de l'exemple 1.

On analyse ces deux fragments ainsi qu'un fil brut de PGA servant à fabriquer la pièce tissée, par microscopie électronique à balayage, par spectroscopie Infra-rouge, ESCA, DSC.
. Microscopie électronique à balayage (MEB)
   L'examen de la surface du fil brut de PGA montre la présence de quelques impuretés et de quelques défauts induits lors de la fabrication et/ou lors de la manipulation. Sur la surface de la pièce avant le traitement selon l'invention, on observe la présence d'impuretés en forme de billes à la surface des fils de la pièce, mais, en général, on observe une couche qui recouvre la surface des fils. Sur la pièce après le traitement selon l'invention, on observe quelques impuretés sans toutefois retrouver la couche d'impuretés recouvrant la surface de la pièce avant le traitement selon l'invention. La couche d'impuretés introduite lors du tissage est donc lavée lors du traitement selon l'invention par le dioxyde de carbone supercritique.
. Analyse XPS (ESCA) :
   Les analyses XPS ont été effectuées uniquement sur les fragments de pièce, le diamètre et la texture du fil brut rendant l'analyse impossible. Des analyses de survol et de haute résolution ont été effectuées sur les deux fragments. Le tableau 1 suivant donne la composition atomique de la surface de ces fragments. 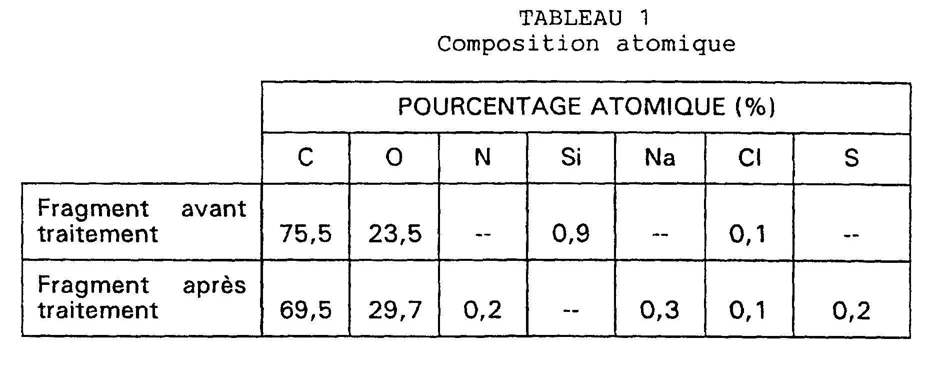
   Ces résultats montrent que le traitement de l'invention entraîne une modification de la composition atomique de surface. En effet, il se produit une variation de la proportion de C et O ainsi que la disparition de Si au profit de N, de Na et de S. De plus, les deux fragments ont une composition de surface qui est éloignée de la composition théorique du PGA (50 % de C et 50 % de O). Ceci montre que la composition chimique de la surface des fragments avant traitement n'est pas formée de PGA pur et que cette composition chimique change suite au traitement selon l'invention.
   Les analyses à haute résolution du carbone (C1s) révèlent aussi que la surface de la pièce est modifiée par le traitement selon l'invention. En effet, le tableau 2 suivant montre que le pourcentage des liaisons C-C et C-O a diminué au profit des liaisons C=O après le traitement. 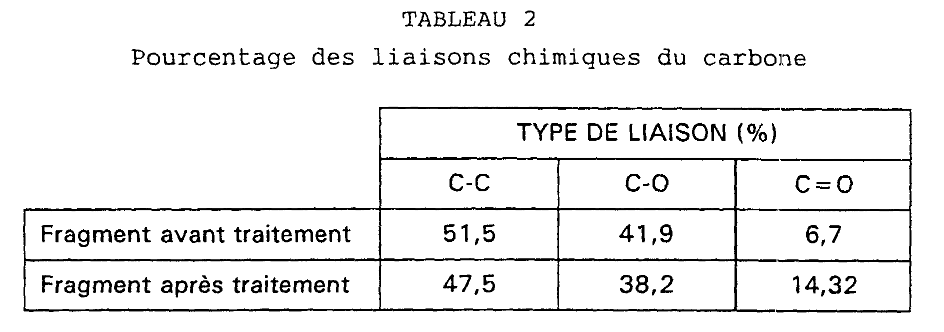
. Analyse FTIR :
   Les spectres infrarouges des deux fragments et du fil brut sont pratiquement identiques sauf pour ce qui est des bandes caractéristiques des groupes CH₂ et CH₃ (entre 2800 et 3100 cm⁻¹). On remarque que la structure des bandes des trois échantillons est différente, et que le fragment avant le traitement a une structure différente de celle des deux autres échantillons. En effet, deux pics importants apparaissent à 2850 cm⁻¹ et 2920 cm⁻¹ après le tissage et puis disparaissent après le traitement selon l'invention. Le spectre du fragment après le traitement est peu différent de celui du fil brut.
. Analyse DSC :
   Le tableau 3 suivant résume les résultats obtenus à partir des thermogrammes. 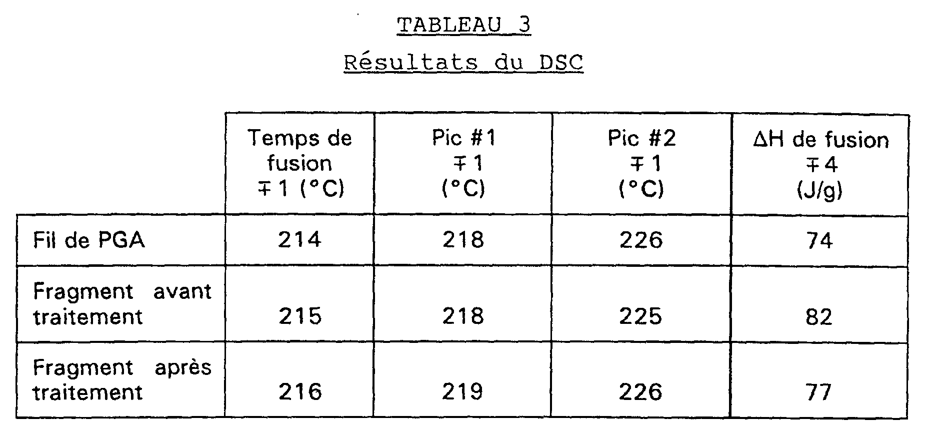
   Il est à noter que les variations de valeur obtenues pour l'enthalpie de fusion ne sont pas significatives, compte tenu des incertitudes de mesure. En conséquence, ces résultats confirment que la structure interne du fil et des fragments de pièce tissée n'est pas modifiée par le traitement de l'invention.

On constate donc d'après toutes ces analyses que :
. le traitement ne modifie pas la structure intrinsèque du second fragment et des fibres de PGA,
. les deux fragments renfermaient des contaminants de type hydrocarbures qui disparaissent dans le second fragment après le traitement selon l'invention,
. la qualité du nettoyage des fibres après le traitement selon l'invention est satisfaisante,
. le traitement induit une modification de la composition chimique de la surface des fils, probablement responsable d'une augmentation de la résistance mécanique.

### EXEMPLE 3

On analyse la viscosité d'un fragment de pièce tissée et traitée selon l'invention comme décrit à l'exemple 1, en la comparant à la viscosité d'un fil brut de PGA 100, et à celle d'un fragment de la même pièce tissée de PGA 100 non traité au dioxyde de carbone supercritique.

La viscosité est un indicateur de la dégradation de la structure textile de la pièce tissée. Plus la viscosité est élevée, plus la masse moléculaire du polymère est élevée et moins la structure textile de la pièce tissée est dégradée.

L'analyse de la viscosité donne les résultats suivants :

| | |
|---|---|
| Fil brut de PGA | 1,09 dl/g |
| Fragment avant traitement | 1,16 dl/g |
| Fragment après traitement selon l'invention | 1,13 dl/g |

Ces résultats montrent que l'étape de tissage ne réduit en rien la viscosité du matériau, mais qu'il en est de même pour le traitement selon l'invention.

### EXEMPLE 4

Des fils de PGA 100 (acide polyglycolique 100 %) ont été traités par un courant de CO₂ supercritique pendant au moins 12 heures à une température de 35° C sous une pression de 2,7.10⁷ à 2,8.10⁷ Pa.

Plusieurs types d'échantillons ont été traités : de simples fils de PGA brut de filage séparés les uns des autres, et des fils de PGA compactés pour former une structure non-tissée.

A l'issue de ce traitement au fluide supercritique, tous les échantillons ont été comparés aux mêmes échantillons avant leur traitement au fluide supercritique. Aucune différence d'aspect, de résistance ou de souplesse n'a été constatée à l'examen visuel et au toucher.

L'invention permettant simultanément de purifier et de renforcer les caractéristiques mécaniques des structures textiles contenant au moins un polymère de la famille des poly(α-hydroxy-acides) trouve des applications avantageuses dans toutes les situations où l'on recherche l'une et/ou l'autre de ces propriétés. En particulier, les pièces tissées de PGA traitées selon l'invention sont pures et biorésorbables et peuvent entrer dans la composition d'implants ou constituer des implants pour l'homme ou l'animal (ligaments artificiels, patch, membranes artificielles, prothèses vasculaires, cardiaques ou osseuses, ...).

## Revendications

1. Procédé de traitement après sa fabrication d'une structure textile contenant au moins un polymère de la famille des poly(α-hydroxy-acides) obtenue par au moins une étape mécanique d'assemblage (filature et/ou tressage et/ou tricotage et/ou tissage et/ou agglomération...) de fibres textiles au moins partiellement formées de fibres contenant une proportion d'au moins un polymère de la famille des poly(α-hydroxy-acides) dont le poids moléculaire est adapté pour permettre l'obtention de ladite structure, procédé dans lequel on soumet la structure à au moins une étape de traitement lors de laquelle on la place au contact d'un courant d'une composition d'au moins un fluide à l'état supercritique, et on choisit ladite composition, et la durée du traitement pour éliminer les impuretés emprisonnées dans la structure afin de permettre son implantation ultérieure dans le corps humain ou animal, et pour au moins préserver l'intégrité de la structure.

2. Procédé selon la revendication 1, **caractérisé en ce qu'**on utilise du dioxyde de carbone à l'état supercritique à titre de composition.

3. Procédé selon la revendication 2, **caractérisé en ce qu'**on place la structure au contact d'un courant de dioxyde de carbone supercritique pendant une durée comprise entre 1 h et 24 h, notamment de l'ordre de 12 h.

4. Procédé de fabrication d'une structure textile contenant au moins un polymère de la famille des poly(α-hydroxy-acides), dans lequel on réalise la structure par au moins une étape mécanique d'assemblage (filature et/ou tressage et/ou tricotage et/ou tissage et/ou agglomération...) de fibres textiles au moins partiellement formées de fibres contenant une proportion d'au moins un polymère de la famille des poly(α-hydroxy-acides) dont le poids moléculaire est adapté pour permettre l'obtention de ladite structure, procédé dans lequel après avoir effectué toutes les étapes d'assemblage permettant d'obtenir la structure, on soumet la structure à au moins une étape de traitement lors de laquelle on la place au contact d'un courant d'une composition d'au moins un fluide à l'état supercritique, et on choisit ladite composition et la durée du traitement pour éliminer les impuretés emprisonnées dans la structure afin de permettre son implantation ultérieure dans le corps humain ou animal, et pour au moins préserver l'intégrité physique de la structure.

5. Procédé selon la revendication 4, **caractérisé en ce qu'**on réalise la structure par assemblage de fils obtenus par extrusion de fibres contenant au moins un polymère de la famille des poly(α-hydroxy-acides).

6. Procédé selon l'une des revendications 4 et 5, **caractérisé en ce qu'**on réalise la structure avec des fils obtenus par extrusion de fibres de PGA.

7. Procédé selon l'une des revendications 4 à 6, **caractérisé en ce qu'**on réalise le conditionnement final de la structure après ladite étape de traitement.

8. Procédé selon l'une des revendications 4 à 7, **caractérisé en ce qu'**on soumet la structure à une et une seule étape de purification après toutes les étapes d'assemblage permettant d'obtenir la structure.

9. Procédé selon l'une des revendications 4 à 8, **caractérisé en ce qu'**on soumet la structure à l'étape de traitement immédiatement après avoir effectué toutes les étapes d'assemblage et de finition pour l'obtention de la structure.

10. Procédé selon l'une des revendications 1 à 9, **caractérisé en ce qu'**on effectue ladite étape de traitement immédiatement après la fabrication de la pièce.

11. Procédé selon l'une des revendications 1 à 10, **caractérisé en ce qu'**on utilise du dioxyde de carbone à l'état supercritique à titre de composition de fluide(s) à l'état supercritique.

12. Procédé selon la revendication 11, **caractérisé en ce qu'**on place la structure au contact d'un courant de dioxyde de carbone supercritique pendant une durée comprise entre 1 h et 24 h, notamment de l'ordre de 12 h.

13. Structure textile biorésorbable contenant au moins un polymère de la famille des poly (α-hydroxy-acides) obtenue par au moins une étape mécanique d'assemblage (filature et/ou tressage et/ou tricotage et/ou tissage et/ou agglomération...) de fibres textiles au moins partiellement formées de fibres contenant une proportion d'au moins un polymère de la famille des poly(α-hydroxy-acides) dont le poids moléculaire est adapté pour permettre l'obtention de ladite structure, et ayant été obtenue par un procédé selon l'une des revendications 1 à 12.

14. Structure selon la revendication 13, **caractérisée en ce qu'**elle est formée d'un assemblage de fils obtenus par extrusion de fibres contenant au moins un polymère de la famille des poly(α-hydroxy-acides).

15. Structure selon l'une des revendications 13 et 14, **caractérisée en ce qu'**elle est formée avec des fils obtenus par extrusion de fibres de PGA.

16. Structure selon l'une des revendications 13 à 15, **caractérisée en ce qu'**elle est formée d'une pièce tissée biorésorbable de fils de PGA.

17. Pièce comprenant une structure textile selon l'une des revendications 13 à 16 suffisamment pure pour pouvoir être implantable chez l'homme ou l'animal.

## Patentansprüche

1. Verfahren zur Behandlung, nach der Herstellung, einer Textilstruktur, die wenigstens ein Polymer der Poly(α-Hydroxycarbonsäuren)-Familie enthält und mit wenigstens einer mechanischen Wirkstufe (Spinnen und/oder Flechten und/oder Stricken und/oder Weben und/oder Anhäufung...) von Textilfasern erhalten wird, die wenigstens teilweise von Fasern gebildet werden, die einen Anteil von wenigstens einem Polymer der Poly(α-Hydroxycarbonsäuren)-Familie enthalten, dessen Molekulargewicht so eingestellt wird, dass die genannte Struktur erhalten werden kann, wobei in dem Verfahren die genannte Struktur wenigstens einer Behandlungsstufe unterzogen wird, bei der sie mit einem Strom einer Zusammensetzung von wenigstens einem Fluid im überkritischen Zustand in Kontakt gebracht wird, und wobei die genannte Zusammensetzung und die Dauer der Behandlung so gewählt werden, dass in der Struktur eingeschlossene Verunreinigungen eliminiert werden, um deren spätere Implantation in einen menschlichen oder tierischen Körper zu gestatten und um die Integrität der Struktur zu erhalten.

2. Verfahren nach Anspruch 1, **dadurch gekennzeichnet, dass** Kohlendioxid im überkritischen Zustand als Zusammensetzung verwendet wird.

3. Verfahren nach Anspruch 2, **dadurch gekennzeichnet, dass** die Struktur für eine Dauer von 1 bis 24 Stunden, insbesondere von etwa 12 Stunden, mit einem Strom aus überkritischem Kohlendioxid in Kontakt gebracht wird.

4. Verfahren zur Herstellung einer Textilstruktur, die wenigstens ein Polymer der Poly(α-Hydroxycarbonsäuren)-Familie enthält, wobei die Struktur mit wenigstens einer mechanischen Wirkstufe (Spinnen und/oder Flechten und/oder Stricken und/oder Weben und/oder Anhäufung...) von Textilfasern erhalten wird, die wenigstens teilweise von Fasern gebildet werden, die einen Anteil von wenigstens einem Polymer der Poly(α-Hydroxycarbonsäuren)-Familie enthalten, dessen Molekulargewicht so eingestellt wird, dass die genannte Struktur erhalten werden kann, wobei in dem Verfahren, nach der Durchführung aller Wirkstufen zum Erhalten der Struktur, die Struktur wenigstens einer Behandlungsstufe unterzogen wird, bei der sie mit einem Strom einer Zusammensetzung von wenigstens einem Fluid im überkritischen Zustand in Kontakt gebracht wird, und wobei die genannte Zusammensetzung und die Dauer der Behandlung so gewählt werden, dass in der Struktur eingeschlossene Verunreinigungen eliminiert werden, um deren spätere Implantation in einen menschlichen oder tierischen Körper zu gestatten und um die physische Integrität der Struktur zu erhalten.

5. Verfahren nach Anspruch 4, **dadurch gekennzeichnet, dass** die Struktur durch Wirken der Fäden realisiert wird, die durch die Extrusion von Fasern erhalten wurden, die wenigstens ein Polymer der Poly(α-Hydroxycarbonsäuren)-Familie enthalten.

6. Verfahren nach Anspruch 4 oder 5, **dadurch gekennzeichnet, dass** die Struktur mit Fäden realisiert wird, die durch die Extrusion von PGA-Fasern erhalten wurden.

7. Verfahren nach einem der Ansprüche 4 bis 6, **dadurch gekennzeichnet, dass** die Endkonditionierung der Struktur nach der genannten Behandlungsstufe erfolgt.

8. Verfahren nach einem der Ansprüche 4 bis 7, **dadurch gekennzeichnet, dass** die Struktur nach allen Wirkstufen zum Erzielen der Struktur einer einzigen Reinigungsstufe unterzogen wird.

9. Verfahren nach einem der Ansprüche 4 bis 8, **dadurch gekennzeichnet, dass** die Struktur unmittelbar nach der Durchführung aller Wirk- und Schlichtstufen zum Erzielen der Struktur der Behandlungsstufe unterzogen wird.

10. Verfahren nach einem der Ansprüche 1 bis 9, **dadurch gekennzeichnet, dass** die genannte Behandlungsstufe unmittelbar nach der Herstellung des Artikels erfolgt.

11. Verfahren nach einem der Ansprüche 1 bis 10, **dadurch gekennzeichnet, dass** Kohlendioxid im überkritischen Zustand als Zusammensetzung von Fluid(en) im überkritischen Zustand verwendet wird.

12. Verfahren nach Anspruch 11, **dadurch gekennzeichnet, dass** die Struktur für eine Dauer von 1 bis 24 Stunden, insbesondere von etwa 12 Stunden, mit einem Strom aus überkritischem Kohlendioxid in Kontakt gebracht wird.

13. Biologisch resorbierbare Textilstruktur, die wenigstens ein Polymer der Poly(α-Hydroxycarbonsäuren)-Familie enthält und die mit wenigstens einer mechanischen Wirkstufe (Spinnen und/oder Flechten und/oder Stricken und/oder Weben und/oder Anhäufung...) von Textilfasern erhalten wird, die wenigstens teilweise von Fasern gebildet werden, die einen Anteil von wenigstens einem Polymer der Poly(α-Hydroxycarbonsäuren)-Familie enthalten, dessen Molekulargewicht so eingestellt wird, dass die genannte Struktur erhalten werden kann, und hergestellt mit einem Verfahren nach einem der Ansprüche 1 bis 12.

14. Struktur nach Anspruch 13, **dadurch gekennzeichnet, dass** sie ein Fadengewirk bildet, die durch die Extrusion von Fasern erhalten wurden, die wenigstens ein Polymer der Poly(α-Hydroxycarbonsäuren)-Familie enthalten.

15. Struktur nach einem der Ansprüche 13 oder 14, **dadurch gekennzeichnet, dass** sie mit Fäden gebildet wird, die durch die Extrusion von PGA-Fasern erhalten wurden.

16. Struktur nach einem der Ansprüche 13 bis 15, **dadurch gekennzeichnet, dass** sie von einem biologisch resorbierbaren Webartikel aus PGA-Fasern gebildet wird.

17. Artikel, umfassend eine Textilstruktur nach einem der Ansprüche 13 bis 16, die rein genug ist, um einem Menschen oder einem Tier implantiert zu werden.

## Claims

1. A process for treating a textile structure, following its manufacture, containing at least one polymer of the poly(α-hydroxy-acids) family, obtained by at least one stage for the mechanical assembly (spinning and/or braiding and/or knitting and/or weaving and/or agglomeration etc) of textile fibres formed at least partially of fibres containing a proportion of at least one polymer of the poly(α-hydroxy-acids) family of which the molecular weight is adapted so as to enable the said structure to be obtained, process wherein the structure is subjected to at least one treatment stage during which it is placed in contact with a flow of a composition of at least one fluid in the supercritical state, and wherein the said composition and the duration of the treatment stage are chosen so as to eliminate impurities trapped within the structure so that the structure can subsequently be implanted in the human body or an animal body and the integrity of the structure is at least preserved.

2. The process as claimed in claim 1, **characterised in that** carbon dioxide in the supercritical state is used as the composition.

3. The process as claimed in claim 2, **characterised in that** the structure is placed in contact with a flow of supercritical carbon dioxide for a period of between 1 h and 24 h, in particular of the order of 12 h.

4. A process for manufacturing a textile structure containing at least one polymer of the poly(α-hydroxy-acids) family, wherein the structure is produced by at least one stage for the mechanical assembly (spinning and/or braiding and/or knitting and/or weaving and/or agglomeration etc) of textile fibres formed at least partially of fibres containing a proportion of at least one polymer of the poly(α-hydroxy-acids) family of which the molecular weight is adapted so as to enable the said structure to be obtained, process wherein after having carried out all the assembly stages enabling the structure to be obtained, the structure is subjected to at least one treatment stage during which it is placed in contact with a flow of a composition of at least one fluid in the supercritical state, and wherein the said composition and the duration of the treatment are chosen so as to eliminate impurities trapped within the structure so that the structure can subsequently be implanted in the human body or an animal body and the physical integrity of the structure is at least preserved.

5. The process as claimed in claim 4, **characterised in that** the structure is produced by assembling threads obtained by extruding fibres containing at least one polymer of the poly(α-hydroxy-acids) family.

6. The process as claimed in either of claims 4 or 5, **characterised in that** the structure is produced with threads obtained by extruding PGA fibres.

7. The process as claimed in one of claims 4 to 6, **characterised in that** the final conditioning of the structure is carried out after the said treatment stage.

8. The process as claimed in one of claims 4 to 7, **characterised in that** the structure is subjected to one and only one purification stage after all the assembly stages enabling the structure to be obtained.

9. The process as claimed in one of claims 4 to 8, **characterised in that** the structure is subjected to the treatment stage immediately after having carried out all the assembly and finishing stages for obtaining the structure.

10. The process as claimed in one of claims 1 to 9, **characterised in that** the said treatment stage is carried out immediately after the piece is manufactured.

11. The process as claimed in one of claims 1 to 10, **characterised in that** carbon dioxide is used in the supercritical state as the fluid composition(s) in the supercritical stage.

12. The process as claimed in claim 11, **characterised in that** the structure is placed in contact with a flow of supercritical carbon dioxide for a period of between 1 h and 24 h, in particular of the order of 12 h.

13. A bioresorbable textile structure containing at least one polymer of the poly(α-hydroxy-acids) family, obtained by at. least one stage for the mechanical assembly (spinning and/or braiding and/or knitting and/or weaving and/or agglomeration etc) of textile fibres formed at least partially of fibres containing a proportion of at least one polymer of the poly (α-hydroxy-acids) family of which the molecular weight is adapted so as to enable the said structure to be obtained, and having been obtained by a process as claimed in one of claims 1 to 12.

14. The structure as claimed in claim 13 **characterised in that** it is formed of an assembly of threads obtained by extruding fibres containing at least one polymer of the poly(α-hydroxy-acids) family.

15. The structure as claimed in either of claims 13 or 14, **characterised in that** it is formed of threads obtained by extruding PGA fibres.

16. The structure as claimed in one of claims 13 to 15, **characterised in that** it is formed of a bioresorbable woven piece made of PGA threads.

17. A piece comprising a textile structure as claimed in one of claims 13 to 16 which is sufficiently pure for it to be implanted in man or in an animal.
